(19) Europäisches Patentamt
European Patent Office
Office européen des brevets

(11) EP 4 001 271 A1

(12) **EUROPEAN PATENT APPLICATION**

(43) Date of publication:
**25.05.2022 Bulletin 2022/21**

(21) Application number: **20207246.8**

(22) Date of filing: **12.11.2020**

(51) International Patent Classification (IPC):
***C07D 401/04*** *(2006.01)* ***A01N 43/56*** *(2006.01)*

(52) Cooperative Patent Classification (CPC):
**C07D 401/04; A01N 43/56**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Bayer Aktiengesellschaft
51373 Leverkusen (DE)**
- **Bayer Animal Health GmbH
51373 Leverkusen (DE)**

(72) Inventors:
- **REMBIAK, Andreas
65812 Bad Soden (DE)**
- **OLENIK, Britta
46242 Bottrop (DE)**
- **KEIL, Birgit
40231 Düsseldorf (DE)**
- **DOCKNER, Michael
50674 Köln (DE)**
- **LÖGERS, Michael
42327 Wuppertal (DE)**
- **MEHL, Benedikt
42109 Wuppertal (DE)**

(74) Representative: **Cohausz & Florack
Patent- & Rechtsanwälte
Partnerschaftsgesellschaft mbB
Bleichstraße 14
40211 Düsseldorf (DE)**

(54) **CRYSTALLINE FORM OF 2-CHLORO-N-CYCLOPROPYL-5-[1-[2,6-DICHLORO-4-[1,2,2,2-TETRAFLUORO-1-(TRIFLUOROMETHYL)ETHYL]PHENYL]PYRAZOL-4-YL]-N-METHYL-PYRIDINE-3-CARBOXAMIDE**

(57) The present invention relates to a crystalline form A of 2-chloro-N-cyclopropyl-5-[1-[2,6-dichloro-4-[1,2,2,2-tetrafluoro-1-(trifluoromethyl)ethyl]phenyl]pyrazol-4-yl]-N-methyl-pyridine-3-carboxamide (compound of formula (I)) and to the process of preparing the crystalline form A of the compound of formula (I).

EP 4 001 271 A1

## Description

The present invention relates to a crystalline form A of 2-chloro-N-cyclopropyl-5-[1-[2,6-dichloro-4-[1,2,2,2-tetrafluoro-1-(trifluoromethyl)ethyl]phenyl]pyrazol-4-yl]-N-methyl-pyridine-3-carboxamide, to the process of preparing said crystalline form A and to its use in agriculture.

2-Chloro-N-cyclopropyl-5-[1-[2,6-dichloro-4-[1,2,2,2-tetrafluoro-1-(trifluoromethyl)ethyl]phenyl]pyrazol-4-yl]-N-methyl-pyridine-3-carboxamide is depicted below, and will also be referred to as compound of formula (I) in the following.

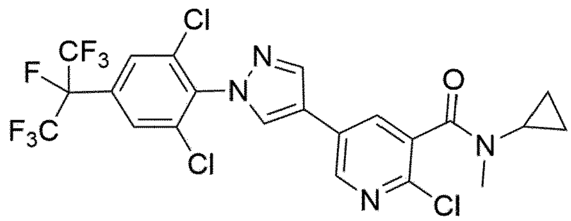

(I)

The compound of formula (I) is associated to be of relevance in the field of pesticides and insecticides. Processes of manufacturing of the amorphous compound are known from e.g. WO2015067646, WO2015067647, WO2016174052, WO2018104214, and WO2019243243. The processes known from the prior art yield the compound of formula (I) in solid form.

Active ingredients may present themselves in their solid states both in amorphous or crystalline forms. Amorphous forms lack a long-range order of its components (like molecules, atoms or ions) while crystalline forms present a high-structured arrangement of these building blocks in form of a crystal lattice.

Polymorphism is the ability of a compound to crystallize in different phases with different arrangements and/or conformations of the molecules in the crystal lattice. Hence, polymorphs are different crystalline forms of the same pure chemical compound. On account of the different arrangement and/or conformation of molecules, amorphous and crystalline forms including polymorphs exhibit different physical, chemical, and biological properties. Properties that may be affected include but are not limited to solubility, dissolution rate, stability, crystal shape, optical and mechanical properties, etc. The thermodynamic stability of amorphous and crystalline forms including polymorphs depends on its free energy.

The occurrence of active ingredients in different solid forms like amorphous forms and crystalline forms is of decisive importance for the production in industrial scale as well as for the development of formulations containing the active substance, as unwanted phase change can lead to thickening and potentially solidification of the formulation and/or large crystals, which can lead to blockages in application equipment, e.g. in spray nozzles in agricultural application machinery. The knowledge of the existence of different solid forms like amorphous or crystalline forms and their properties is thus of high relevance. Nevertheless, it is generally not predictable whether a given chemical compound forms amorphous or crystalline forms, in particular polymorph forms at all and if so, which physical and biological properties these forms may have.

In addition pseudopolymophic forms, named hydrates or solvates, can occur. A solvate is a crystalline molecular compound in which molecules of the solvent of crystallisation are incorporated into the host lattice, consisting of unsolvated molecules. A hydrate is a special case of a solvate, when the incorporated solvent is water. The presence of solvent molecules in the crystal lattice influences the intermolecular interactions and confers unique physical properties to each solvate. A solvate thus has its own characteristic values of internal energy, enthalpy, entropy, Gibbs free energy, and thermodynamic activity.

However, the number of crystalline forms including polymorph forms for active ingredients is highly variable and there is little scientific insights to what determines the number of crystalline forms including polymorph forms. In general, it cannot be predicted how many crystalline forms may occur and in particular not under what conditions.

Further, for active ingredients to give effective biological performance it is important for the respective molecules to get in a solution state to be bioavailable to the target. As an example, this can be achieved on a crop by the presence of dew in the morning on plant leaves, which can slowly dissolve the active ingredient allowing the molecules to distribute over the leaf surface and penetrate inside the leaf. As another example, this can be achieved in soil by rain or irrigation water, which slowly dissolve the active ingredient molecules allowing the molecules to distribute in the soil. The rate of dissolution of crystals depends on the surface area and can be described by the Noyes- Whitney equation:

$$\frac{dm}{dt} = A\frac{D}{d}(C_s - C_t)$$

where m is the mass of dissolved material, t is time and dm/dt is the dissolution rate, D is the diffusion coefficient of the active ingredient in solution, A is the interfacial surface area of the solid, V is the volume of solution, d is the thickness of the diffusion boundary layer, $C_s$ is the concentration of a saturated solution of the active ingredient at the surface of the crystal and $C_t$ is the concentration of the active ingredient in the bulk medium at time t.

For achieving good bioavailability of an active ingredient, it is important for dm/dt to be as high as possible. This can be achieved by increasing the specific surface area A of the crystals, which is the total surface area per unit of mass.

Knowing that different crystalline forms of a compound may differ from each other with respect to one or more physical properties, such as solubility, dissolution rate, stability, crystal shape, optical and mechanical properties, there remains an interest to find crystalline forms of compound (I), which may be advantageous over the amorphous form of compound (I) in terms of some of the above listed properties, e.g. in terms of the stability.

In view of the above, it is an object of the present invention to provide a crystalline form of compound (I). In particular, it is an object of the present invention to provide a crystalline form of compound (I), which possesses sufficient stability.

Further, it is another object of the present invention to provide a process for producing said crystalline form A of compound (I) within short duration times.

At least one of these objects are achieved by the subject matter of the claims.

In one aspect, the present invention relates to a crystalline form A of 2-chloro-N-cyclopropyl-5-[1-[2,6-dichloro-4-[1,2,2,2-tetrafluoro-1-(trifluoromethyl)ethyl]phenyl]pyrazol-4-yl]-N-methyl-pyridine-3-carboxamide (i.e. compound of formula (I)), which in an X-ray powder diffractogram at 25°C and Cu-$K_\alpha$1 radiation, displays at least the following reflections, quoted as 2θ value ± 0.2°: 10.7, 16.2, and 16.7.

In the following, preferred embodiments of the crystalline form A are described in further detail. It is to be understood that each preferred embodiment is relevant on its own as well as in combination with other preferred embodiments.

In a preferred embodiment A1 of the first aspect, an X-ray powder diffractogram at 25°C and Cu-$K_\alpha$1 radiation, displays at least the following reflections, quoted as 2θ value ± 0.2°: 10.7, 16.2, 16.7, 16.9, and 23.2.

In a preferred embodiment A2 of the first aspect, an X-ray powder diffractogram at 25°C and Cu-$K_\alpha$1 radiation, displays at least the following reflections, quoted as 2θ value ± 0.2°: 5.0, 10.7, 16.2, 16.7, 16.9, 23.2, and 23.6; preferably at least the following reflections: 5.0, 10.7, 14.1, 16.2, 16.7, 16.9, 18.5, 22.7, 23.2, and 23.6.

In a preferred embodiment A3 of the first aspect, a Raman spectrum displays at least the following bands, quoted as peak maximum in $cm^{-1}$: 93, 1376, and 1604.

In a preferred embodiment A4 of the first aspect, a Raman spectrum displays at least the following bands, quoted as peak maximum in $cm^{-1}$: 93, 368, 953, 1376, and 1604, preferably at least the following bands: 93, 223, 368, 680, 953, 1376, and 1604, and in particular at least the following bands: 93, 223, 368, 680, 953, 1376, 1604, 2993, 3020, and 3041.

In a preferred embodiment A5 of the first aspect, an Infrared spectrum displays at least the following bands, quoted as peak maximum in $cm^{-1}$: 983, 1214, and 1224.

In a preferred embodiment A6 of the first aspect, an Infrared spectrum displays at least the following bands, quoted as peak maximum in $cm^{-1}$: 726, 737, 983, 1214, and 1224, preferably at least the following bands: 726, 737, 816, 983, 1214, 1224, and 1654, and in particular at least the following bands: 726, 737, 816, 952, 983, 1170, 1214, 1224, 1561, and 1654.

In a second aspect, the present invention relates to a process for the production of the crystalline form A as claimed, comprising the steps of:

a) preparing a solution or a dispersion of a compound of formula (I) being in a form different from the crystalline form A in a solvent selected from aqueous solvents, organic solvents, and mixtures thereof;

b) effecting crystallization of the compound of formula (I), preferably by stirring or shaking the dispersion at a temperature of at least - 5°C; and

c) isolating the resulting precipitate.

In a preferred embodiment B1 of the second aspect, effecting the crystallization in step b) is performed at

stepwise cooling cycles, comprising a first cooling cycle to 70°C, a second cooling cycle to 50°C in a time range from 4 to 10 hours, prefer-ably from 6 to 8 hours, and a third cooling cycle to - 5°C in a time range from 0.25 to 2.5 hours, preferably from 0.5 to 1.5 hours.

In a preferred embodiment B2 of the second aspect, step b) is carried out in the presence of seed crystals of the crystalline form A as claimed.

In a third aspect, the present invention relates to an insecticidal composition, comprising the compound of the formula (I) in the crystalline form A as claimed and optionally an extender and/or a surface-active substance.

In a fourth aspect, the present invention relates to a pesticidal or parasiticidal composition, comprising the compound of the formula (I) in the crystalline form A as claimed and optionally acceptable pesticidal or parasiticidal carriers and/or auxiliaries.

In a fifth aspect, the present invention relates to the crystalline form A according to the first aspect or to the composition according to the fourth aspect for combating parasites in and on animal.

In a sixth aspect, the present invention relates to the crystalline form A according to the first aspect or to the composition according to the third aspect for use as an insecticide.

Further embodiments of the present invention can be found in the claims, the description and the examples. It is to be understood that the features mentioned above and those still to be illustrated below of the subject matter of the invention can be applied not only in the respective given combination but also in other combinations without leaving the scope of the invention.

It has surprisingly been found that, crystalline form A of compound (I) has beneficial physicochemical properties, in particular is advantageous in terms of its storability.

Figures

Figure 1 (Fig. 1): X-ray powder diffractogram of the crystalline form A of the compound of formula (I).

Figure 2 (Fig. 2): FT Raman spectrum of the crystalline form A of the compound of formula (I).

Figure 3 (Fig. 3): IR spectrum of the crystalline form A of the compound of formula (I).

Figure 4 (Fig. 4): Microscopic image of crystal shapes for crystalline form A of the compound of formula (I) obtained by slow evaporation at room temperature with polarized light.

Figure 5 (Fig. 5): Illustration of different crystal shapes with edge length a, b, and c.

Figure 6a (Fig. 6a): Overview of DVS study of the crystalline form A of the compound of formula (I).

Figure 6b (Fig. 6b): Detailed view of DVS study of the crystalline form A of the compound of formula (I).

The terms "compound (I)" and "compound of the formula (I)" are interchangeable and refer to 2-chloro-N-cyclopropyl-5-[1-[2,6-dichloro-4-[1,2,2,2-tetrafluoro-1-(trifluoromethyl)ethyl]phenyl]pyrazol-4-yl]-N-methyl-pyridine-3-carboxamide.

Further, the terms "crystalline form A of the compound of formula (I)", "crystalline form A according to the present invention", "crystalline form A", "crystalline form A as claimed", and the like are interchangeable.

Preferred embodiments regarding the crystalline form A of the compound of formula (I), the process for producing said crystalline form A of the compound of formula (I) and the use thereof are described in detail hereinafter. It is to be understood that the preferred embodiments of the invention are preferred alone or in combination with each other.

The crystalline form A of the compound of formula (I) can be characterized by X-ray powder diffractometry on the basis of the respective diffractograms, which are recorded at 25°C and with Cu-K$\alpha$1 radiation (1.5406 Å).

As indicated above, the present invention relates in one embodiment to a crystalline form A of the compound of formula (I)

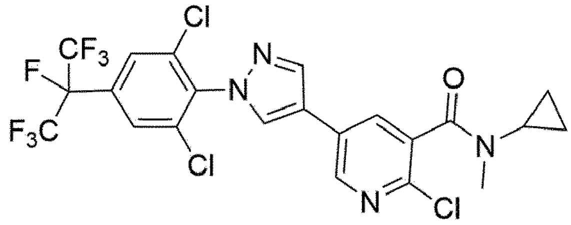

(I)

which in an X-ray powder diffractogram at 25 °C and Cu-$K_\alpha$ 1 radiation, displays at least the following reflections, quoted as 2θ value ± 0.2°: 10.7, 16.2, and 16.7.

According to a preferred embodiment of the present invention, an X-ray powder diffractogram at 25°C and Cu-$K_\alpha$1 radiation, displays at least the following reflections, quoted as 2θ value ± 0.2°: 10.7, 16.2, 16.7, 16.9, and 23.2.

According to a more preferred embodiment of the present invention, an X-ray powder diffractogram at 25°C and Cu-$K_\alpha$1 radiation, displays at least the following reflections, quoted as 2θ value ± 0.2°: 5.0, 10.7, 16.2, 16.7, 16.9, 23.2, and 23.6; even more preferably at least the following reflections: 5.0, 10.7, 14.1, 16.2, 16.7, 16.9, 18.5, 22.7, 23.2, and 23.6.

The crystalline form A according to the present invention displays at least three, often at least five, in particular at least seven, more particularly at least ten, and especially all of the reflections quoted in Table 1 (at 25°C and Cu-$K_\alpha$1 radiation, quoted as 2θ value ± 0.2°).

Table 1: X-ray reflections of the crystalline form A of the compound of formula (I)

| Reflections (Peak maxima) [°2 Theta] |
|---|
| 5.0 |
| 9.9 |
| 10.7 |
| 11.6 |
| 12.0 |
| 12.8 |
| 14.1 |
| 14.2 |
| 14.6 |
| 14.9 |
| 15.3 |
| 16.2 |
| 16.7 |
| 16.9 |
| 17.3 |
| 17.7 |
| 17.9 |
| 18.2 |
| 18.5 |
| 18.8 |
| 19.6 |
| 19.9 |
| 20.0 |

(continued)

| Reflections (Peak maxima) [°2 Theta] |
|---|
| 21.3 |
| 21.8 |
| 22.1 |
| 22.4 |
| 22.7 |
| 23.2 |
| 23.6 |
| 24.1 |
| 24.9 |
| 25.4 |
| 25.8 |
| 26.4 |
| 26.6 |
| 26.9 |
| 27.6 |
| 28.0 |
| 28.2 |
| 28.4 |
| 28.7 |
| 28.9 |
| 29.2 |
| 29.6 |
| 29.9 |
| 30.4 |
| 32.0 |
| 32.9 |
| 33.7 |
| 34.1 |
| 34.3 |
| 35.2 |
| 35.9 |
| 36.7 |
| 37.4 |
| 37.8 |

The crystalline form A of the compound of formula (I) can further be characterized by the X-ray diffractogram depicted in Figure 1.

The crystalline form A of the compound of formula (I) can be characterized by Raman spectroscopy on the basis of the respective spectrum, which are recorded at 25°C and with a resolution of 2 $cm^{-1}$.

According to one embodiment of the present invention, a Raman spectrum displays at least the following bands, quoted as peak maximum in $cm^{-1}$: 93, 1376, and 1604.

According to a preferred embodiment of the present invention, a Raman spectrum displays at least the following bands, quoted as peak maximum in $cm^{-1}$: 93, 368, 953, 1376, and 1604, more preferably at least the following bands: 93, 223, 368, 680, 953, 1376, and 1604, and in particular at least the following bands: 93, 223, 368, 680, 953, 1376, 1604, 2993, 3020, and 3041.

The crystalline form A displays at least three, often at least five, in particular at least seven, and especially all of the bands quoted in Table 2 as peak maxima:

Table 2: Raman bands of the crystalline form A of the compound of formula (I)

| Bands [Peak maxima in $cm^{-1}$] |
|---|
| 3103 |
| 3089 |
| 3041 |
| 3020 |
| 2993 |
| 2943 |
| 1655 |
| 1604 |
| 1566 |
| 1554 |
| 1510 |
| 1433 |
| 1422 |
| 1404 |
| 1392 |
| 1376 |
| 1342 |
| 1281 |
| 1267 |
| 1257 |
| 1239 |
| 1224 |
| 1197 |
| 1187 |
| 1172 |
| 1126 |
| 1111 |
| 1097 |
| 1071 |
| 1044 |
| 1015 |
| 992 |

(continued)

| Bands [Peak maxima in $cm^{-1}$] |
|---|
| 953 |
| 929 |
| 867 |
| 857 |
| 844 |
| 814 |
| 800 |
| 791 |
| 768 |
| 755 |
| 738 |
| 680 |
| 667 |
| 652 |
| 640 |
| 631 |
| 607 |
| 573 |
| 509 |
| 488 |
| 474 |
| 465 |
| 438 |
| 413 |
| 394 |
| 368 |
| 413 |
| 394 |
| 368 |
| 356 |
| 349 |
| 338 |
| 318 |
| 292 |
| 282 |
| 266 |
| 258 |
| 234 |

(continued)

| Bands [Peak maxima in $cm^{-1}$] |
|---|
| 223 |
| 194 |
| 170 |
| 152 |
| 140 |
| 93 |

The crystalline form A of the compound of formula (I) can further be characterized by the Raman spectrum depicted in Figure 2.

The crystalline form A of the compound of formula (I) can be characterized by infrared spectroscopy on the basis of the respective spectrum, which are recorded at 25°C using an universal diamond ATR device and a resolution of 4 $cm^{-1}$.

According to one embodiment of the present invention, an Infrared spectrum displays at least the following bands, quoted as peak maximum in $cm^{-1}$: 983, 1214, and 1224.

According to a preferred embodiment of the present invention, an Infrared spectrum displays at least the following bands, quoted as peak maximum in $cm^{-1}$: 726, 737, 983, 1214, and 1224, preferably at least the following bands: 726, 737, 816, 983, 1214, 1224, and 1654, and in particular at least the following bands: 726, 737, 816, 952, 983, 1170, 1214, 1224, 1561, and 1654.

The crystalline form A display at least three, often at least five, in particular at least seven, and especially all of the bands quoted in the following as peak maxima:

Table 3. IR bands of the crystalline form A of the compound of formula (I)

| Bands [Peak maxima in $cm^{-1}$] |
|---|
| 3120 |
| 3099 |
| 3059 |
| 3042 |
| 3018 |
| 2992 |
| 1710 |
| 1654 |
| 1604 |
| 1561 |
| 1503 |
| 1448 |
| 1432 |
| 1422 |
| 1408 |
| 1402 |
| 1394 |
| 1376 |
| 1362 |

(continued)

| Bands [Peak maxima in $cm^{-1}$] |
|---|
| 1342 |
| 1331 |
| 1306 |
| 1279 |
| 1264 |
| 1254 |
| 1224 |
| 1214 |
| 1199 |
| 1170 |
| 1131 |
| 1119 |
| 1094 |
| 1074 |
| 1064 |
| 1045 |
| 1034 |
| 1013 |
| 983 |
| 952 |
| 929 |
| 920 |
| 888 |
| 868 |
| 856 |
| 843 |
| 816 |
| 800 |
| 792 |
| 767 |
| 756 |
| 737 |
| 726 |
| 682 |
| 667 |
| 652 |
| 637 |
| 630 |

(continued)

| Bands [Peak maxima in $cm^{-1}$] |
|---|
| 609 |
| 572 |
| 565 |

The crystalline form A of the compound of formula (I) can further be characterized by the IR spectrum depicted in Figure 3.

Further, the crystalline form A of the compound of formula (I) shows a needle-like habit (see Fig. 4). A needle-like habit has a larger surface area compared to other crystalline forms (e.g. cubic form). Thus, the crystalline form A of the compound of formula (I) has enhanced dissolution rates resulting in higher bioavailability after application on plant parts, in particular leaves or in the soil.

Figure 5 (i) illustrates schematically a crystal shape with a low aspect ratio and low surface area. Figure 5(ii) illustrates schematically a crystal shape with a high aspect ratio and high surface area for the same mass of material.

As indicated above, the present invention relates in one embodiment to a process for the production of the crystalline form A as defined herein, comprising the steps of:

a) preparing a solution or a dispersion of a compound of formula (I) being in a form different from the crystalline form A in a solvent selected from aqueous solvents, organic solvents, and mixtures thereof;

b) effecting crystallization of the compound of formula (I), preferably by stirring or shaking the dispersion at a temperature of at least - 5°C; and

c) isolating the resulting precipitate.

Suitable solvents in step a) are petroleum ether, hexane, heptane, cyclohexane, methyl-cyclohexane, benzene, toluene, xylene, decalin, chloro-benzene, dichloro-benzene, trifluoromethyl benzene, dichloromethane, chloroform, carbon tetra-chloride, di-chlorethane, tri-chlor- ethane, diethyl ether, diisopropyl ether, methyl tert-butyl -ether, methyl tert-amyl-ether, cyclopentyl-methyl-ether, dioxane, tetrahydrofuran, methyl tetrahydrofuran, 1 ,2-di-methoxyethane, 1, 2-di-ethoxy-ethane, anisole, N,N-dimethyl-formamide, N,N-dimethyl-acetamide, N-methyl-formanilide, acetonitrile, butyronitrile, methanol, ethanol, *iso*-propyl alcohol, 1 -propanol, 2-methoxy ethanol, tert. butanol, 1- butanol, 2-butanol, 3-methyl-1-butanol, propylene carbonate, water, cyclohexanol, ethandiole, ethylene glycol, N-methylpyrrolidone, hexamethylphosphoric -triamide or 1,3-dimethyl-2-2-imidazolinone or N,N- dimethyl acetamide (DMAC), and mixtures thereof.

In one embodiment in step a), the solvent is selected from the group consisting of acetonitrile, ethanol, *iso*-propyl alcohol, 3-methyl-1-butanol, propylene carbonate, water, *n*-heptane, cyclohexane, and mixtures thereof.

In one embodiment effecting the crystallization in step b) is performed via evaporation at a temperature range from 20 to 60°C.

In another embodiment effecting the crystallization in step b) is performed out of a slurry, preferably in a time range from 2 to 10 days. In this connection, the temperature is preferably in a range from 20 to 40°C, more preferably from 20 to 30°C.

In another embodiment effecting the crystallization in step b) is performed at stepwise cooling cycles. The cooling cycles preferably comprise three steps.

The first cooling cycle preferably comprises the step of cooling down to a temperature range from 60 to 80°C, preferably from 65 to 75°C, in particular to 70°C, at a preferred cooling rate range from 0.5 to 3 K/min, more preferably from 1 to 2 K/min, in particular at 1.5 K/min.

The second cooling cycle preferably comprises the step of cooling down to a temperature range from 40 to below 60°C, preferably from 45 to 55°C, in particular to 50°C, in a preferred time range from 4 to 10 hours, more preferably from 6 to 8 hours, in particular in 7 hours.

The third cooling cycle preferably comprises the step of cooling down to a temperature range from - 15 to 5°C, preferably from - 10 to 0°C, in particular to - 5°C, in a preferred time range from 0.25 to 5 hours, more preferably from 0.5 to 1.5 hours or at a preferred cooling rate range from 5 to 15 K/min, more preferably from 8 to 13 K/min, in particular at 10 K/min.

In this connection it is to be understood that between the three cooling steps dwell times may occur, wherein the solution or dispersion can be stirred or is allowed to stand still at the adjusted temperature. The subsequent cooling step may however also follow directly.

In a preferred embodiment, the solution or dispersion obtained after the first cooling cycle is stirred from 0.2 to 3 hours, preferably from 0.5 to 1.5 hours.

In a preferred embodiment effecting the crystallization in step b) is performed at stepwise cooling cycles, comprising a first cooling cycle to a temperature range from 60 to 80°C, preferably from 65 to 75°C, at a preferred cooling rate range from 0.5 to 3 K/min, more preferably from 1 to 2 K/min, a second cooling cycle to a temperature range from 40 to below 60°C, preferably from 45 to 55°C, in a time range from 4 to 10 hours, preferably from 6 to 8 hours, and a third cooling cycle to a temperature range from - 15 to 5°C, preferably from - 10 to 0°C, in a time range from 0.25 to 2.5 hours, preferably from 0.5 to 1.5 hours.

In a particular embodiment effecting the crystallization in step b) is performed at stepwise cooling cycles, comprising a first cooling cycle to 70°C, a second cooling cycle to 50°C in a time range from 4 to 10 hours, preferably from 6 to 8 hours, and a third cooling cycle to - 5°C in a time range from 0.25 to 2.5 hours, preferably from 0.5 to 1.5 hours.

In one embodiment according to the present invention, step b) is carried out in the presence of seed crystals of the crystalline form A as defined herein.

By means of the crystallization, crystalline form A of the compound of formula (I) is obtained with at least 85 %, in particular 90 %, and most preferably at least > 95 % from the herewith disclosed process.

As indicated above, the present invention relates in one embodiment to an insecticidal composition, comprising the compound of the formula (I) in the crystalline form A as disclosed herewith and optionally an extender and/or a surface-active substance.

The insecticidal composition may additionally comprise one or more further active substance(s) selected from the group consisting of herbicides, insecticides, acaricides, fungicides, safeners and/or plant growth regulator. The insecticidal composition may further comprise adjuvants which improve action, such as penetrants, e.g. vegetable oils, for example rapeseed oil, sunflower oil, mineral oils, for example paraffin oils, alkyl esters of vegetable fatty acids, for example rapeseed oil methyl ester or soya oil methyl ester, or alkanol alkoxylates and/or spreaders, for example alkylsiloxanes and/or salts, for example organic or inorganic ammonium or phosphonium salts, for example ammonium sulfate or diammonium hydrogenphosphate and/or retention promoters, for example dioctyl sulfosuccinate or hydroxypropylguar polymers and/or humectants, for example glycerol and/or fertilizers, for example ammonium-, potassium- or phosphorus-containing fertilizers.

The insecticidal composition may comprise auxiliaries such as, for example, extenders, solvents and/or solid carriers and/or other auxiliaries such as, for example, surfactants (also known as surface-active substances). The formulations (i.e. the insecticidal composition) are produced either in suitable facilities or else before or during application.

The auxiliaries used may be substances suitable for imparting special properties, such as certain physical, technical and/or biological properties, to the formulation of the compound of the formula (I), or to the use forms prepared from these formulations (for example ready-to-use pesticides such as spray liquors or seed dressing products).

Suitable extenders are, for example, water, polar and nonpolar organic chemical liquids, for example from the classes of the aromatic and non-aromatic hydrocarbons (such as paraffins, alkylbenzenes, alkylnaphthalenes, chlorobenzenes), the alcohols and polyols (which, if appropriate, may also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulfones and sulfoxides (such as dimethyl sulfoxide). If the extender utilized is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example mineral oil fractions, mineral and vegetable oils, alcohols such as butanol or glycol and their ethers and esters, ketones such as acetone, methyl ethyl ketone, methyl isobutyl ketone or cyclohexanone, strongly polar solvents such as dimethylformamide and dimethyl sulfoxide, and water.

In principle, it is possible to use all suitable solvents. Examples of suitable solvents are aromatic hydrocarbons, such as xylene, toluene or alkylnaphthalenes, chlorinated aromatic or aliphatic hydrocarbons, such as chlorobenzene, chloroethylene or methylene chloride, aliphatic hydrocarbons, such as cyclohexane, paraffins, mineral oil fractions, mineral and vegetable oils, alcohols, such as methanol, ethanol, isopropanol, butanol or glycol and their ethers and esters, ketones such as acetone, methyl ethyl ketone, methyl isobutyl ketone or cyclohexanone, strongly polar solvents, such as dimethyl sulfoxide, and also water.

In principle, it is possible to use all suitable carriers. Useful carriers especially include: for example ammonium salts and ground natural minerals such as kaolins, clays, talc, chalk, quartz, attapulgite, montmorillonite or diatomaceous earth, and ground synthetic minerals such as finely divided silica, alumina and natural or synthetic silicates, resins, waxes and/or solid fertilizers. It is likewise possible to use mixtures of such carriers. Useful carriers for granules include: for example crushed and fractionated natural rocks such as calcite, marble, pumice, sepiolite, dolomite, and synthetic granules of inorganic and organic flours, and also granules of organic material such as sawdust, paper, coconut shells, com cobs and tobacco stalks.

It is also possible to use liquefied gaseous extenders or solvents. Especially suitable are those extenders or

carriers which are gaseous at standard temperature and under atmospheric pressure, for example aerosol propellants such as halogenated hydrocarbons, and also butane, propane, nitrogen and carbon dioxide.

Examples of emulsifiers and/or foam formers, dispersants or wetting agents having ionic or nonionic properties or mixtures of these surfactants are salts of polyacrylic acid, salts of lignosulfonic acid, salts of phenolsulfonic acid or naphthalenesulfonic acid, polycondensates of ethylene oxide with fatty alcohols or with fatty acids or with fatty amines, with substituted phenols (preferably alkylphenols or arylphenols), salts of sulfosuccinic esters, taurine derivatives (preferably alkyl taurates), phosphoric esters of polyethoxylated alcohols or phenols, fatty acid esters of polyols, and derivatives of the compounds containing sulfates, sulfonates and phosphates, for example alkylaryl polyglycol ethers, alkylsulfonates, alkyl sulfates, arylsulfonates, protein hydrolyzates, lignosulfite waste liquors and methylcellulose. The presence of a surfactant is advantageous if one of the compound of the formula (I) and/or one of the inert carriers is insoluble in water and when the application takes place in water.

Further auxiliaries which may be present in the formulations and the use forms derived therefrom nutrients and trace nutrients such as salts of iron, manganese, boron, copper, cobalt, molybdenum and zinc.

Additional components which may be present are stabilizers, such as cold stabilizers, preservatives, antioxidants, light stabilizers, or other agents which improve chemical and/or physical stability. Foam generators or antifoams may also be present.

In addition, the formulations and the use forms derived therefrom may also comprise, as additional auxiliaries, stickers such as carboxymethylcellulose and natural and synthetic polymers in the form of powders, granules or latices, such as gum arabic, polyvinyl alcohol and polyvinyl acetate, or else natural phospholipids such as cephalins and lecithins and synthetic phospholipids.

It is possible if appropriate for still further auxiliaries to be present in the formulations and the use forms derived therefrom. Examples of such additives are fragrances, protective colloids, binders, adhesives, thickeners, thixotropic agents, penetrants, retention promoters, stabilizers, sequestrants, complexing agents, humectants, spreaders. In general, the compound of the formula (I) can be combined with any solid or liquid additive commonly used for formulation purposes.

Useful retention promoters include all those substances which reduce dynamic surface tension, for example dioctyl sulfosuccinate, or increase viscoelasticity, for example hydroxypropylguar polymers.

The crystalline form A has improved formulation properties since e.g. after application onto the plant, plant parts or soil the compound of formula (I) exists as crystalline particles with a needle-like habit. These forms show an increased rate of dissolution from increased surface area with the needle crystal form compared to other forms, e.g. cubic forms.

As indicated above, the present invention further relates in one embodiment to the crystalline form A as disclosed herein or a composition as disclosed herein for use as an insecticide.

In connecting with the herewith disclosed use, it is noted that all plants and plant parts can be treated.

By plants is meant all plants and plant populations such as desirable and undesirable wild plants, cultivars and plant varieties (whether or not protectable by plant variety or plant breeder's rights). Cultivars and plant varieties can be plants obtained by conventional propagation and breeding methods which can be assisted or supplemented by one or more biotechnological methods such as by use of double haploids, protoplast fusion, random and directed mutagenesis, molecular or genetic markers or by bioengineering and genetic engineering methods.

By plant parts is meant all above ground and below ground parts and organs of plants such as shoot, leaf, blossom and root, whereby for example leaves, needles, stems, branches, blossoms, fruiting bodies, fruits and seed as well as roots, conns and rhizomes are listed. Crops and vegetative and generative propagating material, for example cuttings, conns, rhizomes, runners, slips and seeds also belong to plant parts. Preferred plant parts are leaves, roots and seeds.

In order to produce agrochemical formulations, in particular in SC formulations the crystalline forms A of the compound of formula (I) is preferably milled to particle sizes of 0.1 to 50 microns, more preferably 0.5 to 25 microns, and most preferably 1 to 15 microns or 1 to 10 microns.

As indicated above, the present invention relates in one embodiment to a pesticidal or parasiticidal composition, comprising the compound of the formula (I) in the crystalline form A as disclosed herewith and optionally acceptable pesticidal or parasiticidal carriers and/or auxiliaries.

As indicated above, the present invention relates in one embodiment to the crystalline form A as disclosed herein or to a composition as disclosed herein for combating parasites in and on animal.

The animals include agricultural livestock, for example cattle, sheep, goats, horses, pigs, donkeys, camels, buffalo, rabbits, chickens, turkeys, ducks, geese, cultured fish, honey bees. The animals also include domestic animals - also referred to as companion animals - for example dogs, cats, caged birds, aquarium fish, and what are known as test animals, for example hamsters, guinea pigs, rats and mice.

The control of the parasites should reduce cases of death and improve the performance (for meat, milk, wool, hides, eggs, honey etc.) and the health of the host animal, and so the use of the crystalline form A as disclosed herein

or of a composition as disclosed herein enables more economically viable and easier animal husbandry.

In general, the crystalline form A as disclosed herein or the composition as disclosed herein can be employed directly when they are used for the treatment of animals. They are preferably employed in the form of pesticidal or parasiticidal compositions which may comprise the pesticidal or parasiticidal acceptable excipients and/or auxiliaries known in the prior art. In general, such compositions comprise from 0.01 to 98% by weight of active compound, preferably from 0.5 to 90% by weight, based on the total weight of the composition.

The crystalline form A as disclosed herein or the composition as disclosed herein are employed (administered) in a known manner, by enteral administration in the form of, for example, tablets, capsules, potions, drenches, granules, pastes, boluses, the feed-through process and suppositories, by parenteral administration, for example by injection (intramuscular, subcutaneous, intravenous, intraperitoneal inter alia), implants, by nasal administration, by dermal administration in the form, for example, of dipping or bathing, spraying, pouring on and spotting on, washing and powdering, and also with the aid of moulded articles containing the active compound, such as collars, earmarks, tailmarks, limb bands, halters, marking devices, etc. The crystalline form A as disclosed herein or the composition as disclosed herein can be formulated as a shampoo or as suitable formulations applicable in aerosols or unpressurized sprays, for example pump sprays and atomizer sprays.

In the case of employment for livestock, poultry, domestic pets, etc., the crystalline form A as disclosed herein or the composition as disclosed herein can be employed as formulations (for example powders, wettable powders ["WP"], emulsions, emulsifiable concentrates ["EC"], free-flowing compositions, homogeneous solutions and suspension concentrates ["SC"]) or they can be used as a chemical bath. It is to be understood that when crystalline form A is provided in a homogeneous solutions that the homogeneous solution is free of solids, i.e. that the crystalline form A is completely dissolved in said homogeneous solution.

When crystalline form A as disclosed herein or the composition as disclosed herein is provided as formulation, the formulation preferably contains the crystalline form A in an amount of from 1 to 80% by weight, more preferably from 5 to 75% by weight, based on the total weight of the formulation, unless crystalline form A is dissolved. When crystalline form A as disclosed herein or the composition as disclosed herein is provided as homogeneous solution, the compound of formula (I) is comprised in an amount of from 1 to 80% by weight, more preferably from 5 to 75% by weight, based on the total weight of the homogeneous solution.

The following figures and examples further illustrate the present invention.

## Examples

### Methods

All data which is part of the present application has been prepared according to the methods described below unless otherwise indicated. The samples used for measurement were directly used and did not undergo any further sample preparation.

### Microscopy

Microscopic images were taken with the help of a Leica microscope. The samples were placed on a microscope slide in pure silicone oil. They were examined under polarizable light at a magnification of 10 x 1.6 as well as 2.5 x 1, in order to determine their crystallite size and habit.

### Powder X-Ray Diffraction

For Powder X-Ray Diffraction (XRD) the samples were placed into standard glass capillaries (∅ = 0.7 mm). The measurements were performed at room temperature with a D8 Bruker Advance Diffractometer (Cu-K$\alpha$1 = 1.5406 Å, Johansson primary beam monochromator, position sensitive detector) in transmission mode with rotation of the sample. Data were collected in a two-theta range of 2 - 38°. The tube voltage and current were 40 kV and 40 mA, respectively.

Measurement parameters:

| | |
|---|---|
| Anode: | Cu |
| K-Alpha1 [Å]: | 1,54060 |
| Generator: | 40 mA, 40 kV |
| Sample rotation: | Yes |
| Scan axis: | Gonio |

(continued)

| | |
|---|---|
| Starting Position [°2Th.]: | 2.0066 |
| End Position [°2Th.]: | 37.9906 |

Raman spectroscopy

Raman spectra were recorded at room temperature using FT-Raman-spectrophotometers (model RFS 100 and MultiRam) from Bruker. Resolution was 2 $cm^{-1}$. Measurements were performed in glass vials or aluminium discs. There was no sample preparation.

IR- spectroscopy

IR-ATR-spectra were recorded at room temperature using a FT-IR-spectrophotometer one with universal diamond ATR device from Perkin-Elmer. Resolution was 4 $cm^{-1}$. There was no sample preparation.

Differential Scanning Calorimetry

DSC data were obtained on Netzsch Phoenix DSC 204 F1. Approximately 5-15 mg of each sample was placed into a DSC pan and weight accurately recorded. Crimed pans with one pinhole were used for analysis and the samples were heated under nitrogen atmosphere at a rate of 10°C/min.

Thermogravimetric analysis

TG analyses were performed on a Perkin Elmer Thermogravimetric Analyzer Pyris 6 TGA. Approximately 10 - 15 mg of sample was placed in a tared pan and weighted accurately in the TG furnace. The samples were heated in nitrogen at a rate of 10°C/min.

HPLC

- instrument: Agilent 1100
- column: Zorbax SB-C18, 3.5 μm, 150 x 4.6 mm, stainless steel
- oven temperature: 50 °C
- wave length: 210 nm
- flow rate: 2.0 mL / min
- injection volume: 2 μl
- retention time: 5,6 min
- flow agents: A: water + H3PO4 (0.05%)
  B: acetonitrile

Table 4. HPLC conditions

| Time [min] | Solvent A [%] | Solvent B [%] |
|---|---|---|
| 0.0 | 60 | 40 |
| 4.0 | 30 | 70 |
| 10.0 | 10 | 90 |
| 10.5 | 60 | 40 |
| 12.0 | 60 | 40 |

DVS

The water sorption was determined using a DVS Resolution (London, UK) gravimetric sorption analyzer. The sample was dried at 0% relative humidity for 1000 minutes, after which the dry mass was recorded. Humidity was ramped in 10% increments to 90% RH and then back to 0% RH. The equilibrium criterion was set to a relative mass change of dm/dt=0.002%/minute.

Example 1: Preparation of compound of formula (I)

Several possible synthesis of the compound of formula (I) are known. In the following, two exemplary alternatives are presented.

Alternative a)

Preparation of catalyst stock solution:

In a 25 mL 2-necked flask at 21°C under nitrogen 20 mg palladium acetate and 104 mg xantphos were placed in 10 mL THF (8.9 mM) and the yellow solution was stirred under nitrogen at 21°C for 20 min.

Preparation of Arylzinc solution:

In a 500 mL 4-necked flask at 21°C under nitrogen was placed 50.0 g 1-[2,6-dichloro-4-[1,2,2,2-tetrafluoro-1-(trifluoromethyl)ethyl]phenyl]-4-iodo-pyrazole and 200 mL dry toluene was added. The solution was cooled to 0 - 5°C. At this temperature 102.5 mL ethylmagnesium bromide in THF was added over 45 min maintaining the temperature at 0 - 5°C and after 1 min additional stirring a solution of 72.5 mL zinc chloride in THF (0.7M) which was further diluted with 150 mL dry THF, was added over 75min at 0 - 5°C, then warmed to 21°C and stirred at this temperature for additional 10 min.

Reaction set up:

In a 500 mL 4-necked flask at 21°C under nitrogen was placed 28.9 g 5-bromo-2-chloro-N-cyclopropyl-N-methyl-pyridine-3-carboxamide, 50 mL toluene as well as 50 mL THF and the solution was heated to 70°C - 83°C (reflux). To this solution 5.5 mL of the above prepared catalyst stock solution was added. After 1 min the above prepared arylzinc solution was added over 80min keeping the temperature at 70 - 83°C and after full addition the mixture was stirred for additional 2 h at this temperature. HPLC indicates approx. 85 % product. After cooling to 23°C additional 250 mL toluene were added. Then most of the THF was distilled off and the organic phase was washed with 400 mL 10 % HCl. The aqueous phase was back extracted once with 150 mL toluene and afterwards the combined organic fractions were washed with a solution of 10 g N-acetyl cysteine dissolved in 400 mL water. The organic phase was washed with 200 mL water and then concentrated at 40°C / 80 mbar, the residue was suspended in 400 mL n-heptane and the suspension stirred at 60°C for 1 - 2 h. After cooling to 21°C the cream-colored solid was filtrated, washed with 50 mL n-heptane and dried under vacuum at 40°C / 10 mbar.

46.0 g; purity: 99.4 % (Q-HPLC); Pd = 47 ppm (ICP-MS/OES); Zn < 5 ppm (ICP-MS/OES); yield: 80 % compound of formula (I).

Alternative b)

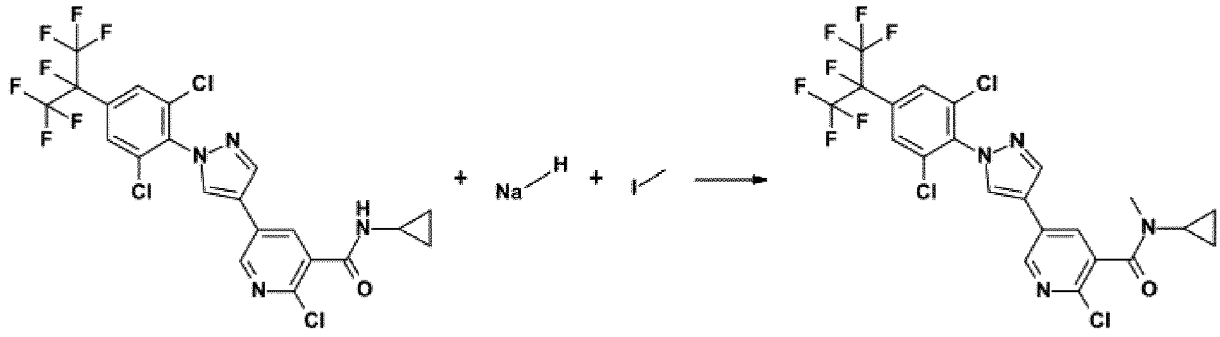

compound (a) compound of formula (I)

Compound (a) is e.g. accessible according to Example I-T3-42 of WO2015067646A1.

To 2.3 liters of THF (over molecular sieve) 11.9 g of NaH (60% in petroleum) was added and the reaction mixture was cooled to 0°C. At 0°C, 143 g of compound (a) was added followed by the dropwise addition of 42.4 g of iodomethane over 15 minutes. The reaction mixture was warmed to ambient temperature and stirred for 2 hours at 15°C. The reaction mixture was poured on 3 liters of ice water and extracted twice with each 1 liter of ethyl acetate. The combined organic phases were extracted twice with each 1.5 liters of sodium chloride solution. The solvent was removed in vacuo to obtain 143.5 g of compound of formula (I) as crude product.

Example 2: Recrystallization

143.5 g of the crude product obtained according to Alternative b) were dissolved in 750 mL of isopropyl alcohol and heated to the boiling point. The solution was cooled to ambient temperature and crystallization was realized over 24 hours at ambient temperature. The crystals were washed with 50 mL of isopropyl alcohol and dried under vacuum at 40 °C to provide crystals of modification A (cf. entry 15 Table 6).

Recrystallization was also realized by dissolving 1550 g crude product as obtained from a synthesis according to Alternative b) in isopropyl alcohol as previously described. After drying the crystals, they were subjected with 10 liters of water and homogenized via an Ultra Turrax. After suction, the product was washed with n-heptane and dried to provide crystals of modification A (cf. entry 16 Table 6).

The solubility of the active provided in Example 1 was further investigated in various solvents (entry 1 to 14 of Table 6). For the determination of the quantitative solubility, the respective solvent(-mixture) was added to approx. 40 mg of the solid, up to a maximum solvent addition of 2 mL in 50 - 200 μL steps. The vials were sealed between each addition of the solvent and the solubility was checked visually. The investigated solution properties are summarized in Table 5. Anti-solvents are those solvents that do not dissolve the active substance completely, after the maximum addition of 2 mL. Optically clear solutions were filtered via a so-called syringe prefilter and evaporated to dryness at different temperatures. Furthermore, cooling crystallizations were carried out. Undissolved substances were stirred for one week at room temperature in the respective anti-solvent.

Table 5. Selection of solvents and anti-solvents

| | Solvent | Anti-solvents |
|---|---|---|
| 1 | isopropyl alcohol | water |
| 2 | propylene carbonate | cyclohexane |
| 3 | ethanol | n-heptane |
| 4 | 3-methyl-1-butanol | |

Based on the solvents and anti-solvents from Table 5, crystallization experiments, listed in Table 6, were carried out to obtain new solid forms of the active provided in Example 1. X-ray powder diffraction was used to assess whether a new phase has been formed.

The cooling crystallization comprised the steps of

1. cooling down to 70°C at 1.5 K/min and stirring for 1 hour at 70°C (500 rpm),

2. cooling down from 70°C to 50°C at 5 K in 7 hours under stirring (500 rpm), and

3. further cooling down from 50°C to - 5°C at 10 K/min under stirring (500 rpm).

Table 6: Recrystallization conditions. "A" denotes the formation of crystalline form A of compound of formula (I); rt denotes room temperature.

| # | solvent | Screening-method | XRD |
|---|---|---|---|
| 1 | isopropyl alcohol (17 mg/mL) | Evaporation ambient conditions | A |
| 2 | propylene carbonate (276 mg/mL) | Slurry - 1 week | A |
| 3 | isopropyl alcohol/ propylene carbonate 65:35 %w/w (268 mg/mL) | Slurry - 1 week | A |
| 4 | isopropyl alcohol/ propylene carbonate 65:35 %w/w (93 mg/mL) | Evaporation 50°C | A |
| 5 | EtOH (25 mg/mL) | Evaporation ambient conditions | amorphous - glassy |
| 6 | $H_2O$ (19 mg/mL) | Slurry - 1 week | A |
| 7 | heptane (19 mg/mL) | Slurry - 1 week | A |
| 8 | cyclohexane (19 mg/mL) | Slurry - 1 week | A |
| 9 | 3-methyl-1-butanol (21 mg/mL) | Evaporation 50°C | amorphous - glassy |
| 10 | 3-methyl-1-butanol | Cooling crystallization | A |
| 11 | 3-methyl-1-butanol/$H_2O$ (99/1) %w/w | Cooling crystallization | A |
| 12 | isopropyl alcohol/$H_2O$ (99/1) %w/w | Cooling crystallization | A |
| 13 | EtOH/$H_2O$ (99/1) %w/w | Cooling crystallization | A |
| 14 | acetonitrile (30 mg/mL) | Evaporation ambient conditions | A |
| 15 | isopropyl alcohol (191 mg/mL) | Ambient conditions | A |
| 16 | isopropyl alcohol and $H_2O$ | Ambient conditions | A |

IR, Raman spectra, X-ray diffractograms (XRD), DSC, and TGA, and HPLC chromatograms were measured for the characterization of the crystalline form A of compound of formula (I). The data for IR, Raman spectra, and X-ray diffractograms (XRD) were obtained from crystals provided by recrystallization according to entry 15 Table 6. The data for DSC, TGA, and HPLC chromatograms were obtained from crystals provided by recrystallization according to entry 16 Table 6.

The results are inter alia depicted in Figure 1 (XRD), Figure 2 (Raman), and Figure 3 (IR). Detailed values of XRD, Raman, and IR can be found in Table 1 to 3 above.

The diffraction pattern of crystalline form A of compound of formula (I) shows the presence of a crystalline substance, indicated by the Bragg reflections (see Fig. 1).

Differential scanning calorimetry (DSC) analysis of crystalline form A has been performed at a heating rate of 10 K/min. The thermogram of crystalline form A disclosed a melting peak with an onset temperature of 175°C. No recrystallisation process was detected. The TG analysis of crystalline form A revealed a mass loss of 0.2 % that coincides with the melting.

The HPLC analysis of crystalline form A showed a purity of 99.5%.

Example 3: Storage

Storage experiments in different environments (25°C, 50°C, 25°C/85 % RH) were performed to investigate the influence of temperature and moisture on the stability of crystalline form A of the compound of formula (I). Based on the comparison to the analysis of the provided material, no phase transition has taken place after twelve weeks in any of

the storage experiments.

a) Influence of temperature

Two samples of crystalline form A were stored at 25°C and 50°C, respectively (see Table 7). The samples were analyzed after 0, 1, 3, and 6 months by DSC, TGA, spectroscopy, X-ray powder diffraction, and HPLC (measurements for chemical stability).

The DCS, TGA, spectroscopy, X-ray powder diffraction, and HPLC measurements did not result in a significant change. Exemplarily, the HPLC results are disclosed in Table 7.

Table 7. Influence of temperature after four, 12, and 24 weeks. Purity determined by HPLC. ACN denotes acetonitrile. *entry 16 of Table 6

| # | Sample used | Storage conditions | Sample Information | Purity [%] | Crystalline Form |
|---|---|---|---|---|---|
| 1 | 16* | - | 1 mg/2mL ACN | 99.54 | A |
| 2 | 16* | 25 °C, 4 weeks | 1 mg/2mL ACN | 98.34 | A |
| 3 | 16* | 25 °C, 4 weeks | 1 mg/2mL ACN | 99.09 | A |
| 4 | 16* | 50 °C, 4 weeks | 1 mg/2mL ACN | 99.11 | A |
| 5 | 16* | 50 °C, 4 weeks | 1 mg/2mL ACN | 99.02 | A |
| 6 | 16* | 25 °C, 12 weeks | 1 mg/2mL ACN | 99.28 | A |
| 7 | 16* | 25 °C, 12 weeks | 1 mg/2mL ACN | 99.23 | A |
| 8 | 16* | 50 °C, 12 weeks | 1 mg/2mL ACN | 99.47 | A |
| 9 | 16* | 50 °C, 12 weeks | 1 mg/2mL ACN | 99.25 | A |
| 10 | 16* | 25 °C, 24 weeks | 1 mg/2mL ACN | 99.40 | A |
| 11 | 16* | 25 °C, 24 weeks | 1 mg/2mL ACN | 99.40 | A |
| 12 | 16* | 50 °C, 24 weeks | 1 mg/2mL ACN | 99.40 | A |
| 13 | 16* | 50 °C, 24 weeks | 1 mg/2mL ACN | 99.34 | A |

b) Influence of Humidity

Two samples of crystalline form A were stored at 25°C/approx. 85 % rel. humidity (see Table 8). The samples were analyzed after 0, 1, 3, and 6 months by DSC, TGA, spectroscopy, X-ray powder diffraction, and HPLC (measurements for chemical stability).

The DCS, TGA, spectroscopy, X-ray powder diffraction, and HPLC measurements did not result in a significant change. Exemplarily, the HPLC results are disclosed in Table 8.

Table 8. Influence of humidity 0, 1, 3 and 6 months. Purity determined by HPLC. ACN denotes acetonitrile. *entry 16 of Table 6

| # | Sample used | Storage conditions [85% RH] | Sample Information | Purity [%] | Crystalline Form |
|---|---|---|---|---|---|
| 1 | 16* | - | 1 mg/2mL ACN | 99.54 | A |
| 2 | 16* | 25 °C, 4 weeks | 1 mg/2mL ACN | 99.02 | A |
| 3 | 16* | 25 °C, 4 weeks | 1 mg/2mL ACN | 99.10 | A |
| 4 | 16* | 25 °C, 12 weeks | 1 mg/2mL ACN | 99.26 | A |
| 5 | 16* | 25 °C, 12 weeks | 1 mg/2mL ACN | 99.25 | A |
| 6 | 16* | 25 °C, 24 weeks | 1 mg/2mL ACN | 99.42 | A |
| 7 | 16* | 25 °C, 24 weeks | 1 mg/2mL ACN | 99.75 | A |

c) Hydrate formation

The DVS study of crystalline form A (cf. entry 16 of Table 6) only shows surface water adsorption (see Figures 6a and 6b). Crystalline form A does not form hydrates and is not hygroscopic. The mass increase over the entire area is negligibly small.

Example 4: Single crystal structure

For determining the single crystal structure of crystalline form A of compound (I), 30 mg of the API was weighed into a 4 mL vial and 1000 μL of acetonitrile were added. The vial was sealed and the mixture was stirred for ten minutes at 25°C with a magnetic stirrer until the solid was completely dissolved. For the evaporation experiment, a syringe needle was used to perforate the PTFE sealing of the vial, which was placed in a fume hood at ambient conditions. After the evaporation experiment was completed, a sample of the crystalline form A of the compound of formula (I) (see Figure 4) was sealed again before further analysis.

A single crystal of sample of the crystalline form A was crystallized from acetonitrile and analyzed by means of single-crystal X-Ray diffraction (SCXRD).

The structure elucidation revealed that crystalline form A crystallizes in a monoclinic unit cell (see Table 9).

Table 9. Crystallographic data

| # | crystalline form A |
|---|---|
| Space group | monoclinic - *P2₁/c* |
| a | 17.8749(4) Å |
| b | 10.8182(2) Å |
| c | 12.4066(3) Å |
| α | 90° |
| β | 94.569(2)° |
| γ | 90° |
| Volume | 2391.49(9) $Å^3$ |
| Final R indices [I>2σ(I)] | R1 = 0.0492, wR2 = 0.1403 |
| R indices (all data) | R1 = 0.0507, wR2 = 0.1420 |
| Goodness of Fit | 1.045 |
| T | 103 (2) K |

## Claims

1. A crystalline form A of the compound of formula (I)

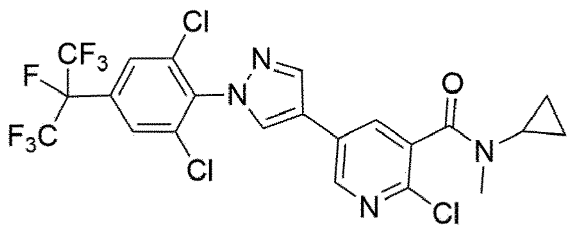
(I)

which in an X-ray powder diffractogram at 25°C and Cu-$K_\alpha$ 1 radiation, displays at least the following reflections, quoted as 2θ value ± 0.2°: 10.7, 16.2, and 16.7.

2. The crystalline form A according to claim 1, which in an X-ray powder diffractogram at 25°C and Cu-$K_\alpha$1 radiation, displays at least the following reflections, quoted as 2θ value ± 0.2°: 10.7, 16.2, 16.7, 16.9, and 23.2.

**3.** The crystalline form A according to claim 1 or 2, which in an X-ray powder diffractogram at 25°C and Cu-$K_\alpha$1 radiation, displays at least the following reflections, quoted as 2$\theta$ value $\pm$ 0.2°: 5.0, 10.7, 16.2, 16.7, 16.9, 23.2, and 23.6; preferably at least the following reflections: 5.0, 10.7, 14.1, 16.2, 16.7, 16.9, 18.5, 22.7, 23.2, and 23.6.

**4.** The crystalline form A according to any one of claims 1 to 3, which in a Raman spectrum displays at least the following bands, quoted as peak maximum in $cm^{-1}$: 93, 1376, and 1604.

**5.** The crystalline form A according to any one of claims 1 to 4, which in a Raman spectrum displays at least the following bands, quoted as peak maximum in $cm^{-1}$: 93, 368, 953, 1376, and 1604, preferably at least the following bands: 93, 223, 368, 680, 953, 1376, and 1604, and in particular at least the following bands: 93, 223, 368, 680, 953, 1376, 1604, 2993, 3020, and 3041.

**6.** The crystalline form A according to any one of claims 1 to 5, which in an Infrared spectrum displays at least the following bands, quoted as peak maximum in $cm^{-1}$: 983, 1214, and 1224.

**7.** The crystalline form A according to any one of claims 1 to 6, which in an Infrared spectrum displays at least the following bands, quoted as peak maximum in $cm^{-1}$: 726, 737, 983, 1214, and 1224, preferably at least the following bands: 726, 737, 816, 983, 1214, 1224, and 1654, and in particular at least the following bands: 726, 737, 816, 952, 983, 1170, 1214, 1224, 1561, and 1654.

**8.** A process for the production of the crystalline form A according to any one of claims 1 to 7, comprising the steps of:

a) preparing a solution or a dispersion of a compound of formula (I) being in a form different from the crystalline form A in a solvent selected from aqueous solvents, organic solvents, and mixtures thereof;
b) effecting crystallization of the compound of formula (I), preferably by stirring or shaking the dispersion at a temperature of at least - 5°C; and
c) isolating the resulting precipitate.

**9.** The process according to claim 8, wherein effecting the crystallization in step b) is performed at stepwise cooling cycles, comprising a first cooling cycle to 70°C, a second cooling cycle to 50°C in a time range from 4 to 10 hours, preferably from 6 to 8 hours, and a third cooling cycle to - 5°C in a time range from 0.25 to 2.5 hours, preferably from 0.5 to 1.5 hours.

**10.** The process according to claim 8 or 9, wherein step b) is carried out in the presence of seed crystals of the crystalline form A according to any one of claims 1 to 7.

**11.** Insecticidal composition, comprising the compound of the formula (I) in the crystalline form A according to any one of claims 1 to 7 and optionally an extender and/or a surface-active substance.

**12.** Pesticidal or parasiticidal composition, comprising the compound of the formula (I) in the crystalline form A according to any one of claims 1 to 7 and optionally acceptable pesticidal or parasiticidal carriers and/or auxiliaries.

**13.** The crystalline form A according to any one of claims 1 to 7 or a composition according to claim 12 for combating parasites in and on animal.

**14.** The crystalline form A according to any one of claims 1 to 7 or a composition according to claim 11 for use as an insecticide.

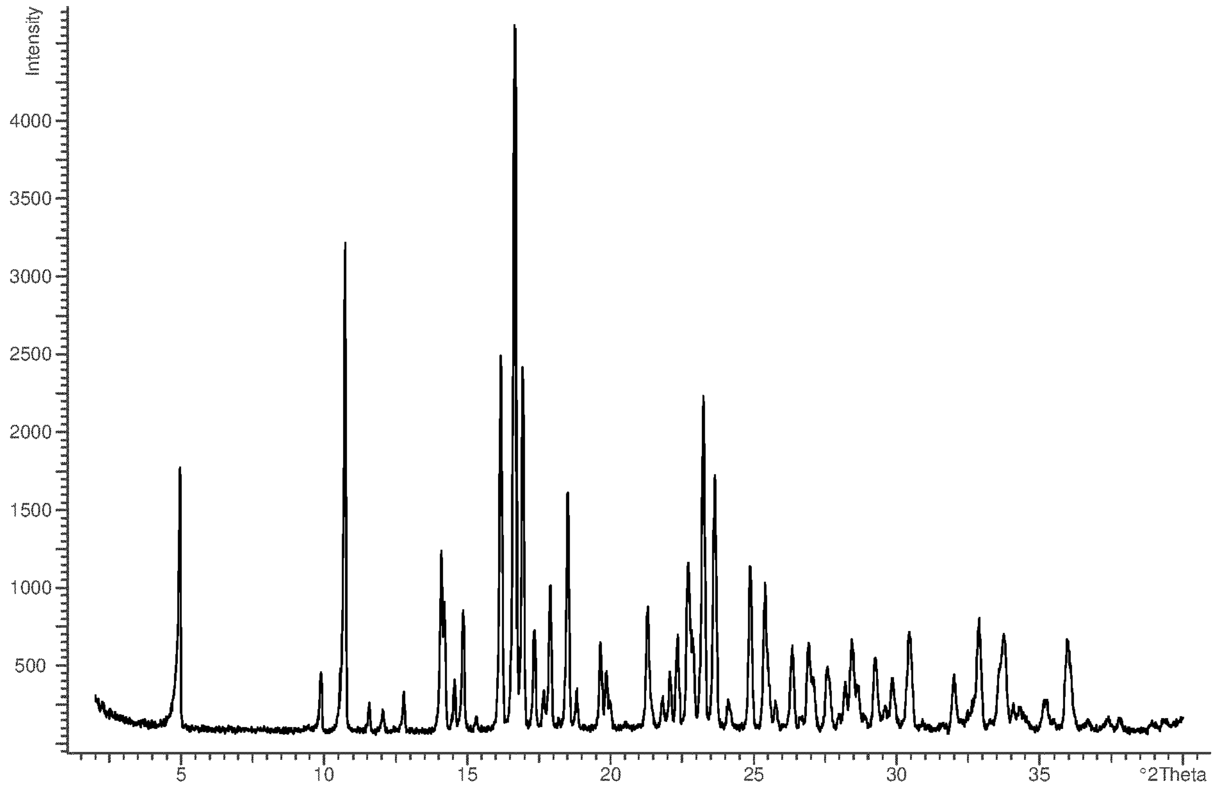
Intensity
4000
3500
3000
2500
2000
1500
1000
500
5
10
15
20
25
30
35
°2Theta

Fig. 1

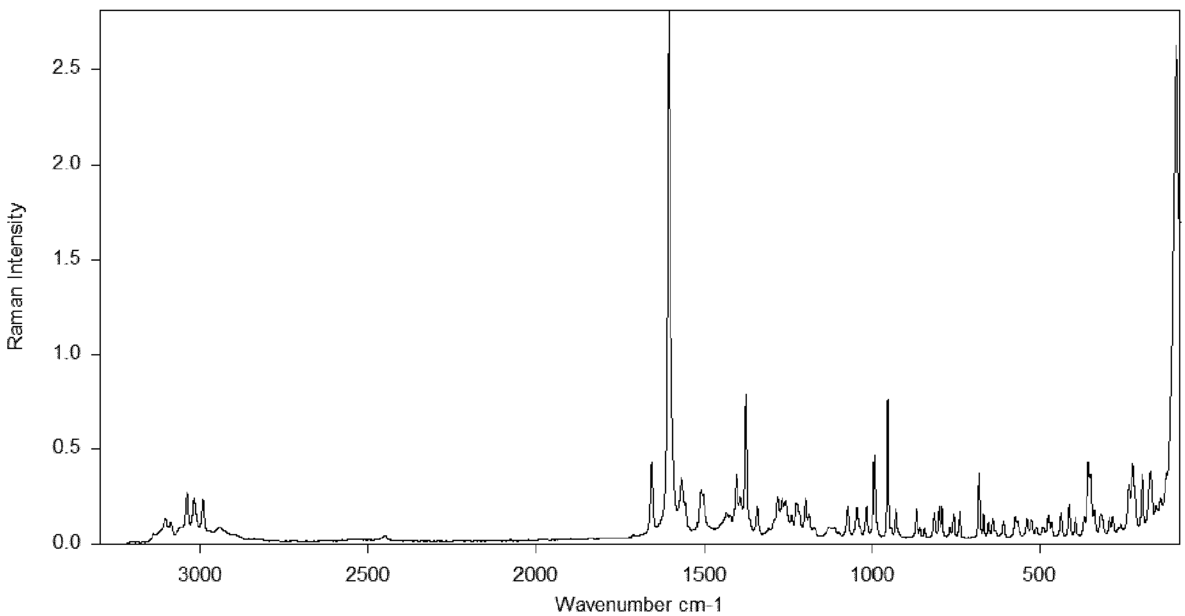
Raman Intensity
2.5
2.0
1.5
1.0
0.5
0.0
3000
2500
2000
1500
1000
500
Wavenumber cm-1

Fig. 2

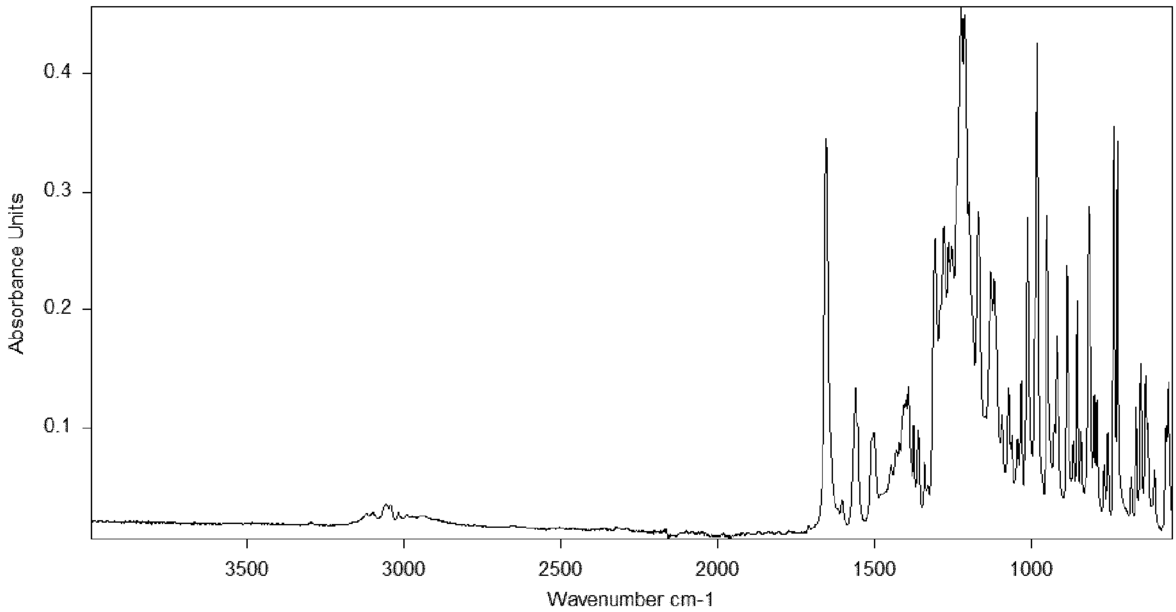
0.4
0.3
0.2
0.1
Absorbance Units
3500
3000
2500
2000
1500
1000
Wavenumber cm-1

Fig. 3

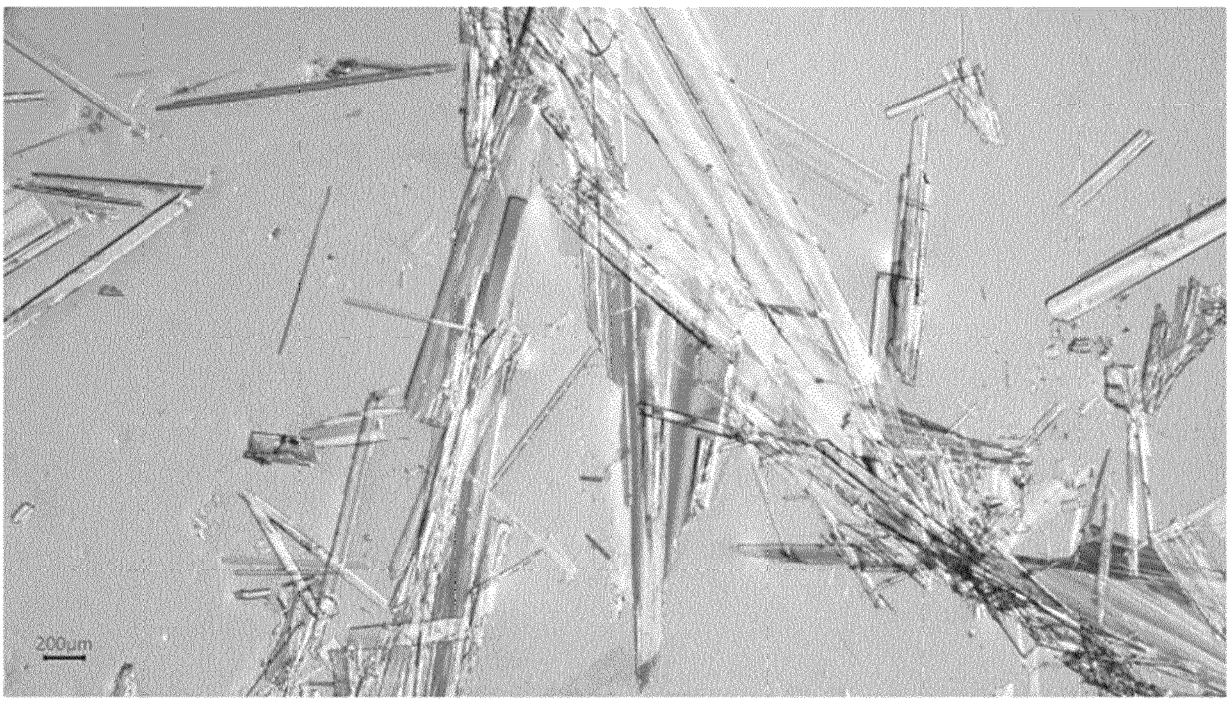
200µm

Fig. 4

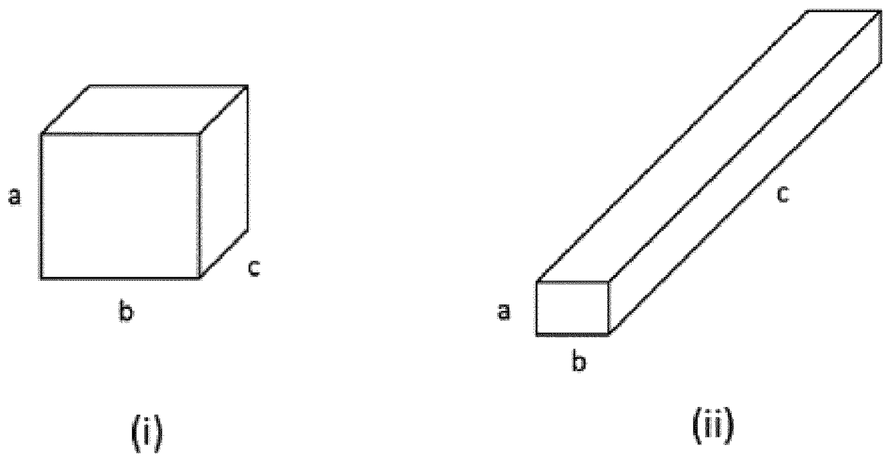
a
b
c
(i)
a
b
c
(ii)

Fig. 5

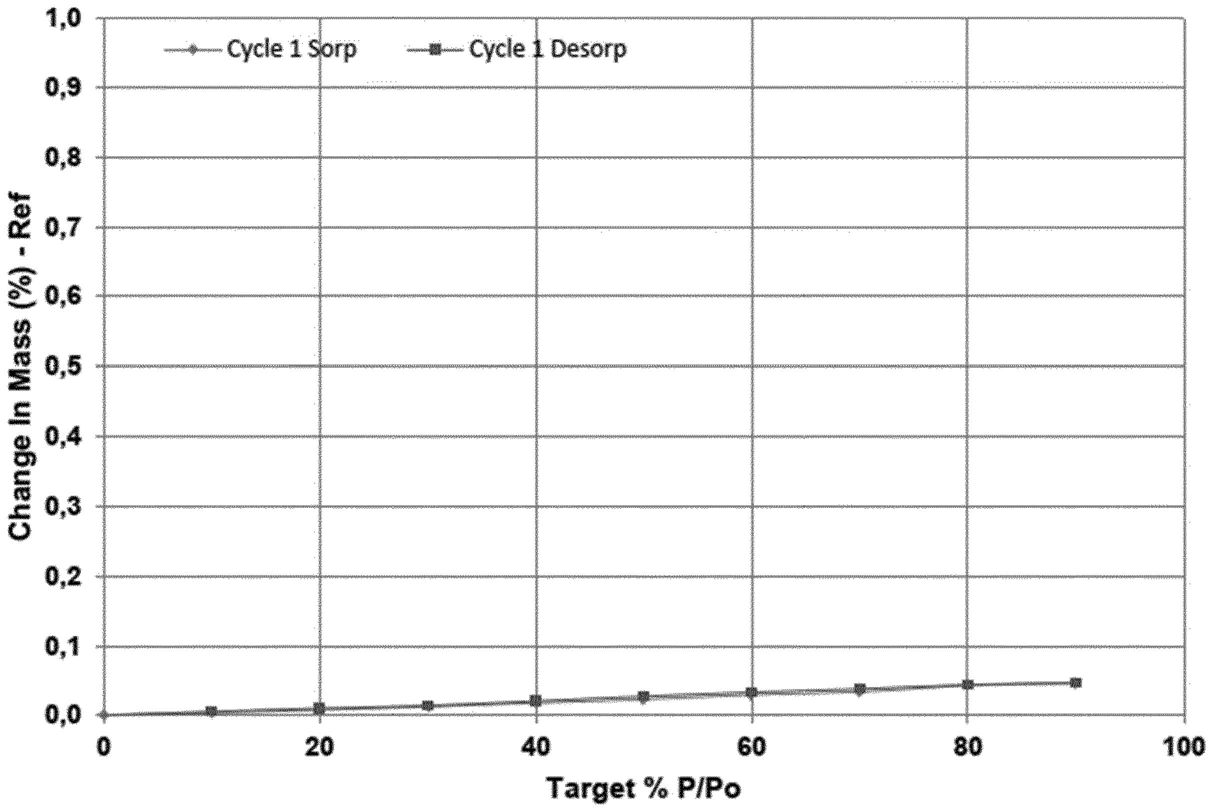
Cycle 1 Sorp
Cycle 1 Desorp
Change In Mass (%) - Ref
Target % P/Po
1,0
0,9
0,8
0,7
0,6
0,5
0,4
0,3
0,2
0,1
0,0
0
20
40
60
80
100

Fig. 6a

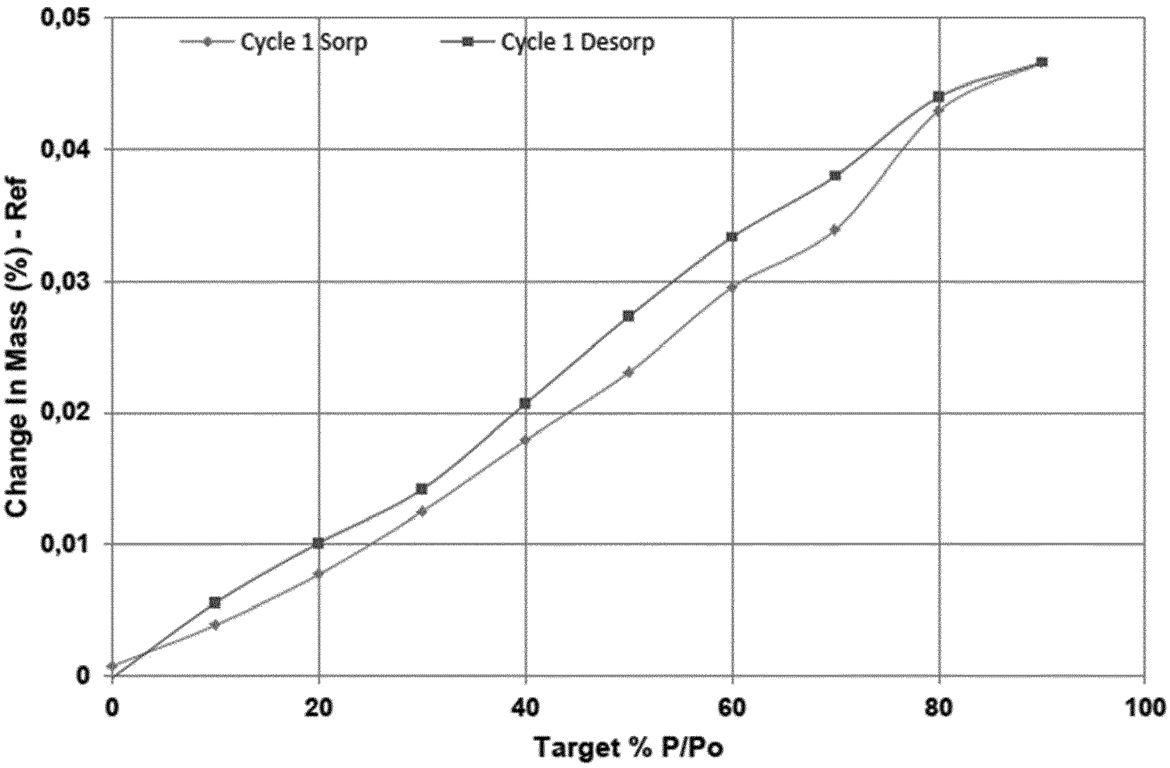
Cycle 1 Sorp
Cycle 1 Desorp
0,05
0,04
0,03
0,02
0,01
0
Change In Mass (%) - Ref
0
20
40
60
80
100
Target % P/Po

Fig. 6b

Europäisches Patentamt
European Patent Office
Office européen des brevets

# EUROPEAN SEARCH REPORT

Application Number
EP 20 20 7246

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/067646 A1 (BAYER CROPSCIENCE AG [DE]) 14 May 2015 (2015-05-14) * page 229, compound I-T3-100; paragraphs [0084], [0739] - [0744]; claims 1-14 * ----- | 1-14 | INV. C07D401/04 A01N43/56 |
| A | EP 3 586 630 A1 (BAYER AG [DE]) 1 January 2020 (2020-01-01) * claims 1-11 * ----- | 1-14 | |

TECHNICAL FIELDS SEARCHED (IPC)
C07D
A01N

The present search report has been drawn up for all claims

1

| Place of search | Date of completion of the search | Examiner |
|---|---|---|
| Munich | 6 April 2021 | Kleidernigg, Oliver |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 20 20 7246

This annex lists the patent family members relating to the patent documents cited in the above-mentioned European search report. The members are as contained in the European Patent Office EDP file on
The European Patent Office is in no way liable for these particulars which are merely given for the purpose of information.

06-04-2021

| Patent document cited in search report | Publication date | Patent family member(s) | Publication date |
|---|---|---|---|
| WO 2015067646 A1 | 14-05-2015 | AU 2014345593 A1 | 26-05-2016 |
| | | AU 2014345594 A1 | 26-05-2016 |
| | | BR 112016010026 B1 | 24-09-2020 |
| | | BR 112016010094 B1 | 29-09-2020 |
| | | CA 2929390 A1 | 14-05-2015 |
| | | CA 2929393 A1 | 14-05-2015 |
| | | CL 2016001079 A1 | 20-01-2017 |
| | | CL 2016001080 A1 | 06-01-2017 |
| | | CN 105873906 A | 17-08-2016 |
| | | CN 106103414 A | 09-11-2016 |
| | | CR 20160209 A | 28-07-2016 |
| | | CR 20160210 A | 14-10-2016 |
| | | CU 20160061 A7 | 02-02-2017 |
| | | DK 3066079 T3 | 13-08-2018 |
| | | DK 3066080 T3 | 13-08-2018 |
| | | DO P2016000103 A | 31-05-2016 |
| | | DO P2016000104 A | 15-06-2016 |
| | | EP 3066079 A1 | 14-09-2016 |
| | | EP 3066080 A1 | 14-09-2016 |
| | | ES 2683443 T3 | 26-09-2018 |
| | | ES 2683445 T3 | 26-09-2018 |
| | | HK 1223099 A1 | 21-07-2017 |
| | | HR P20181290 T1 | 19-10-2018 |
| | | HR P20181296 T1 | 05-10-2018 |
| | | HU E038663 T2 | 28-12-2018 |
| | | HU E038665 T2 | 28-12-2018 |
| | | IL 245353 A | 31-12-2019 |
| | | IL 245408 A | 28-11-2019 |
| | | JP 6840802 B2 | 10-03-2021 |
| | | JP 6840803 B2 | 10-03-2021 |
| | | JP 2016536363 A | 24-11-2016 |
| | | JP 2016536364 A | 24-11-2016 |
| | | JP 2020011964 A | 23-01-2020 |
| | | JP 2020015738 A | 30-01-2020 |
| | | KR 20160079097 A | 05-07-2016 |
| | | KR 20160079851 A | 06-07-2016 |
| | | LT 3066079 T | 10-09-2018 |
| | | LT 3066080 T | 10-09-2018 |
| | | MX 360637 B | 12-11-2018 |
| | | MX 367300 B | 14-08-2019 |
| | | PE 20160905 A1 | 24-09-2016 |
| | | PE 20161343 A1 | 10-12-2016 |
| | | PH 12016500829 A1 | 13-06-2016 |
| | | PH 12016500830 A1 | 13-06-2016 |
| | | PL 3066079 T3 | 31-10-2018 |
| | | PL 3066080 T3 | 31-10-2018 |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO. EP 20 20 7246

This annex lists the patent family members relating to the patent documents cited in the above-mentioned European search report. The members are as contained in the European Patent Office EDP file on
The European Patent Office is in no way liable for these particulars which are merely given for the purpose of information.

06-04-2021

| Patent document cited in search report | Publication date | Patent family member(s) | Publication date |
|---|---|---|---|
| | | PT 3066079 T | 03-10-2018 |
| | | PT 3066080 T | 03-10-2018 |
| | | RU 2016122093 A | 11-12-2017 |
| | | RU 2016122094 A | 11-12-2017 |
| | | SI 3066079 T1 | 28-09-2018 |
| | | SI 3066080 T1 | 30-10-2018 |
| | | SV 2016005194 A | 05-12-2018 |
| | | TR 201808466 T4 | 23-07-2018 |
| | | TR 201808573 T4 | 23-07-2018 |
| | | TW 201609638 A | 16-03-2016 |
| | | TW 201609639 A | 16-03-2016 |
| | | UA 119972 C2 | 10-09-2019 |
| | | UA 120041 C2 | 25-09-2019 |
| | | US 2016278379 A1 | 29-09-2016 |
| | | US 2016297765 A1 | 13-10-2016 |
| | | UY 35819 A | 29-05-2015 |
| | | WO 2015067646 A1 | 14-05-2015 |
| | | WO 2015067647 A1 | 14-05-2015 |
| EP 3586630 A1 | 01-01-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015067646 A
- WO 2015067647 A
- WO 2016174052 A
- WO 2018104214 A
- WO 2019243243 A
- WO 2015067646 A1